# EUROPÄISCHE PATENTSCHRIFT

(11) **EP 2 568 280 B1**
(45) Veröffentlichungstag und Bekanntmachung des Hinweises auf die Patenterteilung: **01.11.2017**
(21) Anmeldenummer: 12183726.4
(22) Anmeldetag: 10.09.2012
(51) Int. Cl.: G01N 27/20, G01N 33/44

(54) **Vorrichtung und Verfahren zur Prüfung von Klebeverbindungen**
Apparatus and method for testing of adhesive joints
Dispositif et méthode pour contrôler des joints adhésifs

(30) Priorität: 09.09.2011 DE 102011082425
(43) Veröffentlichungstag der Anmeldung: 13.03.2013
(73) Patentinhaber: Hochschule für nachhaltige Entwicklung Eberswalde (FH), 16225 Eberswalde (DE)
(72) Erfinder: Schwarz, Ulrich, 16225 Eberswalde (DE); Winkler, Christoph, 13127 Berlin (DE)
(74) Vertreter: Kailuweit & Uhlemann Patentanwälte Partnerschaft mbB

(56) Entgegenhaltungen:
- DE-A1-102007 004 953
- DE-A1-102007 007 617
- DE-A1-102010 002 447
- US-A- 5 245 293
- US-B2- 7 589 457
- BIN SU ET AL: "Fatigue behavior of electrically conductive adhesives", JOURNAL OF ADHESION SCIENCE AND TECHNOLOGY, ZEIST, NL, Bd. 22, Nr. 8-9, 1. Januar 2008 (2008-01-01), Seiten 927-946, XP009165702, ISSN: 0169-4243, DOI: 10.1163/156856108X305516

## Beschreibung

Die Erfindung bezieht sich auf eine Vorrichtung und ein Verfahren zum Structural Health Monitoring von Klebeverbindungen, also eine permanente Kontrolle des Zustandes der Klebeverbindung aufgrund ständiger und/oder regelmäßig und/oder stochastisch wiederholter Messungen.

Bei tragenden Konstruktionen haben Hersteller und Nutzer der Konstruktion ein Bedürfnis den Zustand hinsichtlich seiner Tragfähigkeit zu Überwachen und damit beurteilen zu können. Methodisch sind hier verschiedene Lösungen bekannt: Dehnungsmesstreifen, Längenmessungen, Winkelmessungen Photogrammetrie etc. Diese Methoden sind adaptiv und vermessen in der Regel eine Formänderung.

Weiterhin wird in der US 7,589,457 A1 ein Verfahren zur Überprüfung von Klebeverbindungen vorgestellt, wobei dem Klebstoff piezoelektrisches Material, wie beispielsweise Kristallpartikel, keramische Partikel, Polymerpartikel oder Halbleiterpartikel, zugesetzt werden. Alternativ wird vorgeschlagen dem Klebstoff ein Pulver aus leitfähigem Material, wie z.B. Kupfer, Silber, Eisen, Kohlenstoff oder Ruß, beizumischen. Zur Überprüfung des Zustandes Klebverbindung werden die Fügepartner kontaktiert und die Spannung bzw. der Stromfluss gemessen. Nachteiligerweise ist der messbare Effekt dieser Verfahren eher gering und das Verfahren nur für leitfähige Fügepartner geeignet.

BIN SU ET AL: "Fatigue behavior of electrically conductive adhesives", JOURNAL OF ADHESION SCIENCE AND TECHNOLOGY 22 (2008) 927-946 beschreibt das Ermüdungsverhalten von leitfähigen Klebern, die aus einer isotropen Mischung von Epoxidharz mit Silberpartikeln besteht. Ermittelt wurde eine Zunahme des Widerstandes von mehr als 100% bevor Rißspuren sichtbar werden. Zielrichtung der Studie ist der Einsatz leitfähiger Klebstoffe in der Halbleiterindustrie. Die Untersuchungen wurden mit Klebeflächen auf Biegebalken vorgenommen. Nicht untersucht wurde der Klebstoffeinsatz in einer tatsächlich strukturell belasteten Klebefuge.

Die US 5,245,293 erfasst Änderungen der Eigenschaften geklebter Verbindungen. Überwacht werden dabei dielektrische Eigenschaften mittels eines Mikrodielektrometers.

Insbesondere soll das Eindringen von Feuchtigkeit in die Klebefuge erfasst werden. Untersuchungen unter Biegebeanspruchung bzw. die Detektion daraus resultierender Ermüdungseffekte oder die Abgrenzung von feuchtigkeits- und belastungsinduzierten Degradationen gegeneinander wird nicht thematisiert.

Die DE 10 2010 002 447 A1 beschreibt ein Verfahren und eine Vorrichtung zur Überprüfung der Qualität von Klebeverbindungen zwischen elektronischen Bauteilen. Das verwendete Klebstoffmaterial ist elektrisch leitend und enthält Kohlenstoffnanoröhrchen die beim Klebevorgang über Scherung gezielt ausgerichtet werden können.

Es ist die Aufgabenstellung der vorliegenden Erfindung ein alternatives Verfahren zum Structural Health Monitoring vorzuschlagen, das einen gut messbaren Effekt erzielt und auch für nichtleitende Fügepartner geeignet ist.

Nachfolgend wird das erfindungsgemäße Verfahren zum Structural Health Monitoring mindestens einer Klebefuge beschrieben. Der Klebstoff in der Klebefuge enthält dabei Carbon-Nano-Tubes (CNTs oder Kohlenstoffnanoröhrchen). Beispiele anderer Zusatzstoffe des Klebstoffs, die jedoch nicht unter den Schutzumfang der Ansprüche fallen, sind leitfähige Partikel, bevorzugt metallische Partikel (Au, Ag, Al, Cu, etc.), Ruß, Graphit oder Kohlefasern. Erfindungsgemäß werden die Fügepartner an den für die Verbindung vorgesehenen Flächen mit mindestens teilweise mit leitfähigen Oberflächen versehen, die bekannte Eigenschaften, insbesondere Abmessungen und elektrischen Widerstand, aufweisen.

Erfindungsgemäß werden folgende Schritte durchgeführt:
In einem ersten Schritt a) erfolgt die Kontaktierung der Klebefuge und die elektrische Verbindung mit einer Messeinrichtung zur Messung elektrischer Kenngrößen.

Im darauf folgenden Schritt b) erfolgt die Messung einer elektrischen Kenngröße der Klebefuge. Die Kenngröße ist dabei der elektrische Widerstand der Klebefuge. In einem Schritt c) erfolgt der Vergleich der gemessenen Kenngröße mit einer zuvor ermittelten Referenzgröße. Die Referenzkenngröße wurde dabei zuvor in Versuchen mit einem vergleichbaren Bauteil, wie dem zu überwachenden, ermittelt. Durch Bruchversuche können dabei die Referenzwerte beim Versagen des Bauteils ermittelt werden. Alternativ dazu kann für das gefügte Bauteil durch einen nicht zerstörenden mechanischen Lasteintrag, der im elastischen Bereich der Fügepartner und des Klebstoffs liegt, ein spezifischer Wert bzw. eine Kennlinie mechanischer Eigenschaften (z. B. Festigkeit, Steifigkeit etc.) ermittelt werden.

In einem Schritt d) wird dann der Zustand der zu überwachenden Klebefuge anhand des Ergebnisses des Vergleichs aus Schritt c) bewertet. Die Carbon-Nano-Tubes werden in ihrer Lage bezogen auf die Fügepartner vor dem Abbinden bzw. dem Aushärten des Klebstoffes ausgerichtet. Dadurch kann der zu messende Effekt noch verstärkt werden. Dabei erfolgt die Ausrichtung, indem nach dem Klebstoffauftrag, dieser einem Magnetfeld ausgesetzt wird, wodurch sich die Nano-Tubes in ihrer Lage ausrichten. Darüber hinaus kann auch eine Ausrichtung in einem elektrischen Feld oder in einem elektrischen Feld bei Anwesenheit eines Magnetfeldes erfolgen. Vorzugsweise wird die Feldeinwirkung so lang aufrecht erhalten, bis die Zähigkeit des Klebstoffs soweit angestiegen ist, dass die Carbon-Nano-Tubes nach dem Abschalten der Felder ihre Lage nicht mehr verändern (bspw. durch das Erdmagnetfeld oder sonstige im Raum vorhandene elektrische oder magnetische Felder).Weiterhin bevorzugt erfolgt im Schritt a) die Kontaktierung der Klebefuge mittels in die Fuge eingelegter flächiger, leitfähiger Elemente, bevorzugt Kupferfolien oder Drahtgitter. Bevorzugt sind auch leitfähige Beschichtungen mit einem leitfähigen Beschichtungsmaterial bspw. Kupfer, Zinn, Aluminium, Silber o. ä. (bspw. durch Bedampfen, Pinsel- oder Sprühauftrag oder andere Verfahren aus dem Stand der Technik) der Fügepartner möglich. So werden die für die Verbindung vorgesehenen Flächen wenigstens teilweise mit leitfähigen Oberflächenoberflächen versehen. Diese leitfähigen Oberflächen können dabei den gesamten oder nur einen Teil der für die Verbindung vorgesehenen Flächen bedecken. Es sind auch Muster von leitfähigen Flächen möglich, die vorteilhaft zusammenhängen oder außerhalb der Verbindungsfläche elektrisch kontaktiert sind. Durch nicht zusammenhängende leitfähige Oberflächen auf jedem der Fügepartner ist es möglich, mehrere verschiedene Messverfahren gleichzeitig anzuwenden.

Äußerst vorteilhaft ist somit das Monitoring nicht leitfähiger Fügepartner möglich. Zudem ist das Einlegen der flächigen leitfähigen Elemente sehr einfach und kostengünstig. Besonders geeignet ist das Verfahren zum Monitoring von Klebefugen in Schichtholz. Hier sind Konstruktionsbalken aus Schichtholz besonders interessant, da diese häufig bei Sporthallen und dergleichen Einsatz finden. Beispielsweise bei hohen Schneelasten kann es zum Versagen der Klebefugen kommen, was durch das erfindungsgemäße Verfahren schon im Vorfeld erkannt werden kann.

Bevorzugt werden die Messdaten online an ein Kontrollzentrum übermittelt, wo die Auswertung erfolgt. Vorteilhaft kann so geschultes Personal kostengünstig mehrere Messstellen von einem Kontrollzentrum überwachen. Dabei werden mehrere Klebefugen eines Schichtholzbalkens kontaktiert, wodurch vorteilhaft der gesamte Balken bzw. eine kritische Stelle oder mehrere kritische Stellen überwacht wird bzw. werden, was vor dem Hintergrund, dass Holz als Naturwerkstoff keine homogenen Eigenschaften aufweist, die Sicherheit deutlich erhöht

Besonders bevorzugt wird die Klebefuge derart kontaktiert, dass mindestens eine elektrische Größe im Bereich der zu erwartenden maximalen Beanspruchung gemessen wird. Beispielsweise bei Konstruktionsbalken ist der Ort der maximalen Beanspruchung durch Abschätzung oder Berechnung einfach zu bestimmen. Bei gezielter Messung in diesem Bereich kann der zu messender Effekt erhöht werden.

Die erfindungsgemäße Vorrichtung zur Durchführung des Verfahrens definiert durch die unabhängigen Ansprüche, ist gekennzeichnet durch einen Klebstoff in den Klebefugen eines Schichtholzes, der Carbon-Nano-Tubes enthält und dadurch, dass die Klebefuge elektrisch mit einer Messeinrichtung verbunden ist. Dabei ist die gemessene Kenngröße der elektrische Widerstand und die Messeinrichtung ein Ohmmeter. Die beschriebene Messanordnung kann sich der messtechnischen Wahrnehmung piezoelektrischer Effekte nicht entziehen, da bei ausreichend großen Klebflächen im Fall kurzzeitiger Lastereignisse eine Spannung induziert wird, die über die Widerstandsmessung mit erfasst wird.

Bei der Messung ist die gegenseitige Beeinflussung verschiedener Effekte (z. B. Piezoelektrizität und Widerstand) zu berücksichtigen.

Bevorzugt enthält der Klebstoff einen Anteil von 0,1 bis 10 Masse-% Carbon-Nano-Tubes. Mit einem Anteil in diesem Bereich ist der zu messende Effekt sehr gut ausgeprägt.

Bevorzugt weisen die die Carbon-Nano-Tubes folgende Größe (äußerer Durchmesser) auf: 2 - 200 nm. Vorteilhaft ist der Effekt bei Tubes in diesem Größenbereich besonders gut messbar. Die Länge der Röhren ist dabei sekundär.

Kommen andere Partikel zum Einsatz, die bis auf CNTs nicht unter den Schutzumfang der unabhängigen Ansprüche fallen, so sind folgende Materialien und Konzentrationen (M-% oder Masse-% - Einheit für Masseprozent) bevorzugt:

**Tabelle 1: Zuschlagstoffe**

| Füllstoff | Anteile [M-%] |
|---|---|
| Silber und Gold (Plättchen, Flocken, Nanopartikel), Nickel, Kupfer, Kohlenstoff, silberbeschichtete Glaspartikel, goldbeschichtete Polystyrolkugeln | 0,1 bis 10 M-% |
| Graphitfaser-Vliese, Metall, metallisierte Partikel | 0,1 bis 15 M-% |
| Ruß, Graphit, Eisenoxid-, Kupfer- und Aluminiumteilchen und Aluminiumflocken, metallisierte Glaskugeln oder - fasern, Edelstahl- und Kohlenstofffasern | 0,1 bis 15 M-% |
| Leitfähigkeitsruß, Kohlenstofffasern, Aluminium-Plättchen, gemahlenes Kupfer- & Bronzepulver, rostfreie Stahlfasern, silberbeschichtete Glasplättchen, Silikatkugeln, nickelbeschichtete C-Fasern, Glimmer | 0,1 bis 15 M-% |
| CNT's | 0,1 bis 15 M-% |

Diese Partikel weisen bevorzugt eine Größe von 10 nm bis 0,1 mm und besonders bevorzugt von 50 nm bis 50 µm auf. Es handelt sich um herkömmlich klassierte, handelsübliche Pulvermischungen mit kontinuierlichem Korngrößenspektrum in der jeweiligen Klasse und Schwerpunkt bei der angegebenen Korngröße). Bestimmt werden die Partikelabmessungen vorteilhaft mit der Laserbeugungstechnologie (bspw. Gerät Mastersizer der Fa. Malvern oder LS13320 von Beckman Coulter) oder unter dem Rasterelektronenmikroskop.

Bevorzugt ist der Klebstoff ein Polyurethan-Klebstoff oder ein Melamin-Harnstoff-Formaldehyd-Klebstoff. Es sind jedoch auch Epoxidharz-, Ressorcin-, Isocyanat- Harnstoff-Melamin-Formaldehyd-, Harnstoff-Formaldehyd-, Kasein-, PvAC-, Ethylen-Vinyl-Acetat-, Polyolefinklebstoffe u. a. geeignet.

Nachfolgend wird ein Ausführungsbeispiel der Erfindung erläutert:
Z. B. Brettschichtholz kann mit derartigen Klebstoffen versehen werden. Dies erfolgt in verschiedenen Anordnungen. Das Ausführungsbeispiel wird mit Zeichnungen illustriert, wobei diese im Einzelnen darstellen:

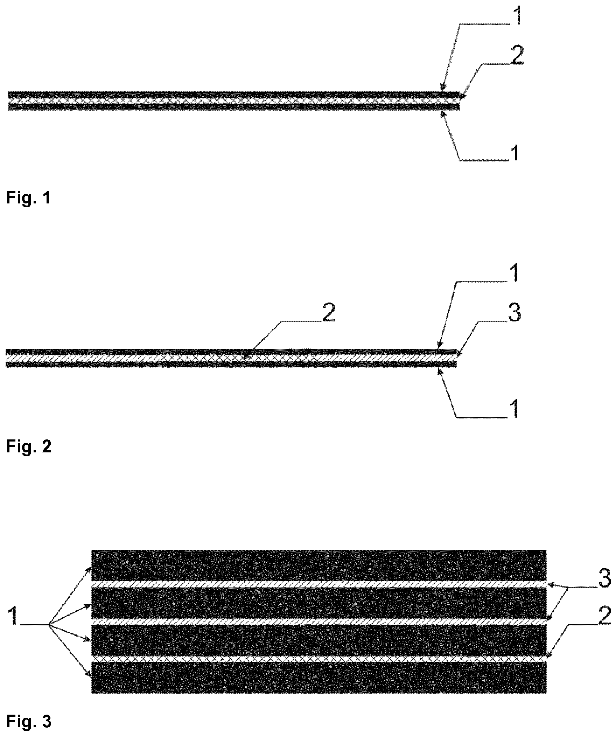

   - Fig. 1: vollflächige Klebverbindung mit zwei Fügepartnern (1) ausgeführt mit dem beschriebenen Klebstoff (2)
   - Fig. 2: teilflächige Klebverbindung (3) mit zwei Fügepartnern (1) ausgeführt mit beschriebenen Klebstoff (2)
   - Fig. 3: Klebverbindung mit mehreren Fügepartnern (1) und einer vollflächig ausgeführten Klebefuge mit beschriebenen Klebstoff (2) sowie nicht meßtechnisch wirksamen vollflächigen Klebefugen (3)
   - Fig. 4: Klebverbindung mit mehreren Fügepartnern (1) und zwei teilflächig ausgeführten Klebefugen mit dem beschriebenen Klebstoff (2) sowie nicht meßtechnisch wirksamen vollflächigen Klebefugen (3)
   - Fig. 5: Klebverbindung mit mehreren Fügepartnern (1) und zwei teilflächig ausgeführten Klebefugen mit dem beschriebenen Klebstoff (2) sowie nicht meßtechnisch wirksamen vollflächigen Klebefugen (3)
   - Fig. 6: Prüfanordnung nach DIN 52186 - 1978
   - Fig. 7: Widerstandsänderung in der unteren Klebefuge (Zugzone) für die Kombination PU mit Ruß
   - Fig. 8: die Widerstandsänderung in der oberen Klebefuge (Druckzone) für die Kombination PU mit Ruß
   - Fig. 9: Geprüfte Lastfälle: Stufenbelastung, Impuls nicht aufgesetzt, Impuls aufgesetzt

Im ausgeführten Zustand ist z. B. die Messung von Biegespannungen in einer geklebten Brettschichtholzkonstruktion möglich. Daneben lassen sich Querkräfte, die z. B. aus wechselnden Lasten resultieren erfassen und hinsichtlich ihrer Relevanz auf das Bauteil bzw. auf die gesamte Konstruktion auswerten.

Für Konstruktionen ist es von Vorteil den aktuellen Zustand einer Struktur bezüglich des mechanischen Zustands diagnostizieren zu können. In Verbindung mit Systemen, die eine Fernüberwachung zu lassen, bieten derartige Systeme ein hohes Maß an Sicherheit. Zusätzlich werden in Verbindung mit der Messung von Belastungsdaten neue Erkenntnisse hinsichtlich des Reaktionsverhaltens gewonnen. Zu eigen ist allen Methoden des *Structural Health Monitoring,* dass diese zerstörungsfrei arbeiten. Ein weiterer Aspekt ist, dass der "Messefühler" die Bauteile hinsichtlich ihrer Eigenschaften nicht beeinflussen darf. Im Wesentlichen sind hier vier Aspekte, die den Betreiber von Konstruktionen interessieren zu nennen:
- Umwelt- und Betriebslasten,
- mechanische Schäden, die durch die oben genannten Lasten hervorgerufen wurden,
- Quantität des Schadens im Betrieb,
- perspektivische Aussagen zu dem weiteren Schadensverlauf.

Über diese Aspekte hinaus geht der Ansatz Bauteilen, die sich von vornherein nicht, beziehungsweise nur bedingt hinsichtlich ihrer mechanischen Eigenschaften beschreiben lassen, so auszustatten, dass eben dies - die genaue Charakterisierung des Bauteils - möglich wird. Alle anderen oben genannten Aspekte bleiben davon unberührt. Z. B. dem Holzbau würde dadurch ein Messinstrument an die Hand gegeben werden, mit dem Konstruktionen erstellt werden können, die auf den Bauteildaten basieren, die auch verbaut wurden. Zusätzlich wäre es möglich, eine laufende Überwachung der Konstruktion durchzuführen. Aus dieser Situation wird es möglich, materialgerechte Lösungen auszuführen, die daneben eben so viel Material, wie dies aus konstruktiver Sicht notwendig ist.

Anhand von Proben aus Buchenholz (andere Holzarten sind ebenfalls möglich, Holz einen Isolator darstellt) wurden standardmäßige Klebsysteme z. B. PU-Klebsysteme, die für konstruktive Zwecke geeignet sind, mit Carbon-Nano-Tubes (z. B. S-MWNT-1020) ausgestattet. Insbesondere ist bei einer Verwendung der so modifizierten Klebstoffe in einem geklebten Prüfkörper mit schichtweisem Aufbau, bei dem die Klebefuge nicht in der neutralen Zone liegt, innerhalb der Klebefuge eine Änderung der elektrischen Eigenschaften z. B. des Ohm'schen Widerstands, in Abhängigkeit der Belastung messbar.

Vorteilhaft haben die CNTs bei kurzzeitigen und langfristigen Belastung erwiesen.

Bevorzugt werden dem Klebstoff noch Hilfsstoffe hinzugefügt, die die Dispergierung der Zuschlagstoffe erleichtern. Die so modifizierten Klebsysteme wurden nach Herstellung der Klebstoff-Zuschlagstoff-Dispersionen verarbeitet. Eingesetzt wurden dabei Lamellen verschiedener Materialien (organisch, metallisch, mineralisch). Jeweils mehrere Lamellen wurden zu einem Prüfkörper mit den oben beschriebenen Klebstoffen gefügt. An den Enden, respektive an den Stirnseiten der Prüfkörper, wurden jeweils zwei Kontaktstellen, z. B. durch Kupferfolien (Kontaktfläche = Klebfugenbreite* ca. 0,05mm*ca. 5mm; elektrische Leitfähigkeit: 58 bis 110 A/(V*m)hergestellt. Sowohl die Auftragsmengen als auch die Pressparameter wurden nach den Vorgaben der Klebstoffhersteller (Datenblätter) durchgeführt.

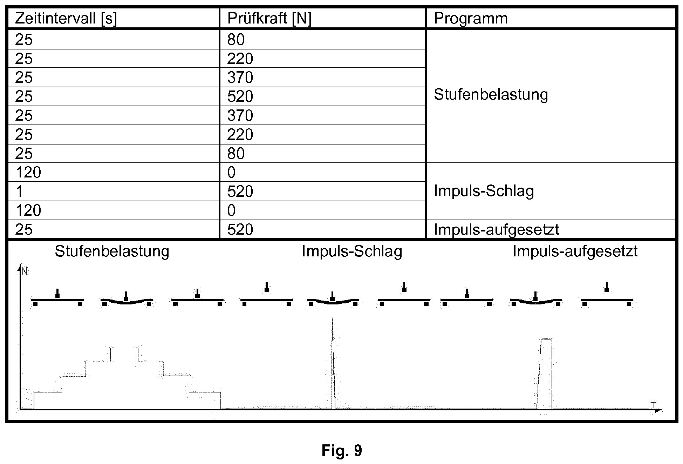

Um die o. g. elektrischen Eigenschaften in Abhängigkeit vermessen zu können, wurde ein spezielles Prüfprogramm entwickelt bei dem z. B. der Ohm'sche Widerstand in der Klebefuge vermessen wurde (siehe Fig. 9, exemplarische Lasten). Im Rahmen der Prüfung wurden verschiedene Laststufen angefahren. Gemessen wurden dabei die Widerstandsänderung in der geklebten Fuge sowie die Durchbiegung des Prüfkörpers.

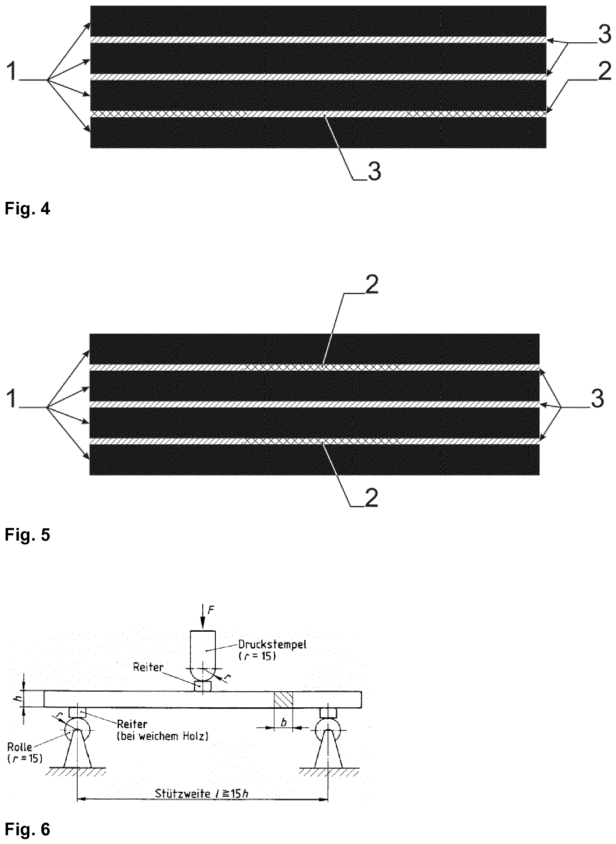

Angelehnt wurde dabei die Prüfung an die DIN 52186 - 1978. Um die elektrischen Verhältnisse und deren Einflüsse weitgehend ausschließen zu können, wurde eine spezielle Prüfmaschine und den elektrischen Verhältnissen angepasste Prüfeinrichtungen konstruiert und gebaut (siehe Fig. 6: Prüfanordnung nach DIN 52186 - 1978).

### Ergebnisse

Mit den verwendeten Zuschlagstoffen (siehe Tabelle 1) in den verschiedenen Klebstoffen zeigt sich ein sehr differenziertes Bild bei den gemessenen Widerständen in Abhängigkeit der Belastung.

Einflussgrößen dabei sind:
- differierende Dicke der geklebten Fuge,
- unterschiedliche Eindringtiefe der Klebstoffe in die Poren des Holzes,
- schwankende Verteilung der Zuschlagstoffen im Klebstoff,
- sensible Messtechnik zur Erfassung der Widerstände in den Klebefugen,
- unzureichende Morphologie der Zuschlagstoffe;

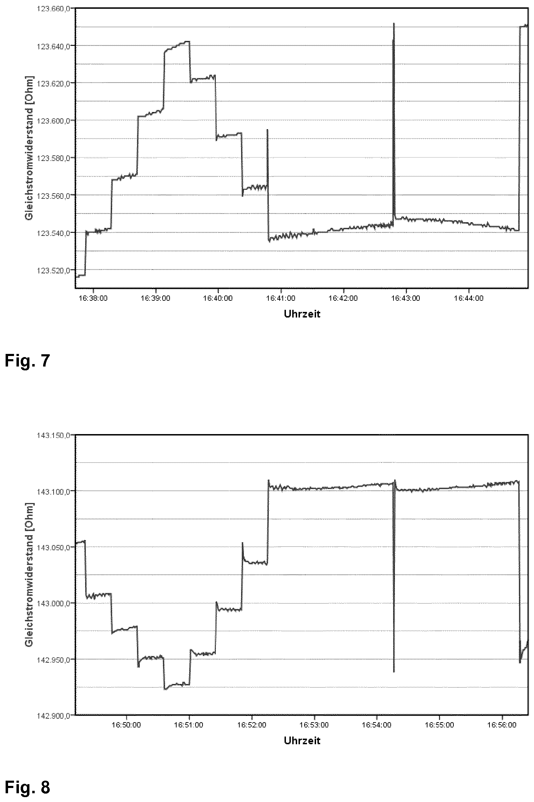

Für die verschiedenen Klebstoff-Zuschlagstoff-Dispersionen wurden eindeutige und stets reproduzierbare Ergebnisse erreicht. Mit dem PU-Klebstoff stellten sich die in Fig.7 und Fig. 8 dargestellten Ergebnisse ein - Zunahme des elektrischen Widerstands innerhalb der Klebefuge, die in der Zugzone liegt sowie eine Verringerung des elektrischen Widerstands in der Druckzone. Exemplarisch dargestellt ist der Verlauf für einen Versuch in den Abbildungen Fig. 7 und Fig. 8.

Eine mögliche Interpretation lässt die Annahme zu, dass im Bereich der Zugbelastungen des Biegebalkens eine Verlängerung und evtl. eine Verbreiterung der Klebefuge stattfindet. Damit würde sich im Sinne der Widerstandsmessung ein Leiter mit einem geringeren Querschnitt ergeben. Daraus resultierend wäre ein höherer Widerstand messbar. Für den Bereich der Druckspannungen in dem Biegebalken ist festzuhalten, dass hier genau der umgekehrte Fall eintritt und eine Stauchung der Klebefuge erfolgt. Damit würde hier eine Verdichtung des Klebstoffes eintreten, womit eine Erhöhung der zur Verfügung stehenden Strompfade eintreten würde.

### Bezugszeichenliste

- 1: Fügepartner
- 2: Klebstoff mit erfindungsgemäßen CNT Zuschlägen
- 3: herkömmlicher Klebstoff

## Patentansprüche

1. Verfahren zum Structural Health Monitoring mehrerer Klebefugen eines Schichtholzes, wobei der Klebstoff (2) in den Klebefugen zwischen jeweils zwei Fügepartnern (1) Carbon-Nano-Tubes enthält, aufweisend folgende Schritte:
a) Kontaktierung der Klebefugen mit einer Messeinrichtung zu Messung des elektrischen Widerstandes der Klebefugen,
b) Messung der elektrischen Widerstände der Klebefugen,
c) Vergleich des gemessenen elektrischen Widerstände der Klebefugen mit zuvor ermittelten Referenzgrößen,
d) Zustandsbestimmung der zu überwachenden Klebefugen anhand des Vergleichs aus Schritt c) **dadurch gekennzeichnet, dass** vor dem Abbinden bzw. dem Aushärten des Klebstoffes (2) die Carbon-Nano-Tubes mittels eines Magnetfeldes,
eines elektrischen Feldes oder eines elektrischen Feldes bei Anwesenheit eines magnetischen Feldes in ihrer Lage bezogen auf die Fügepartner (1) ausgerichtet werden.

2. Verfahren zum Structural Health Monitoring mehrerer Klebefugen eines Schichtholzes nach Anspruch 1, **dadurch gekennzeichnet, dass** im Schritt a) die Kontaktierung der Klebefugen mittels in die Fuge eingelegter flächiger, leitfähiger Elemente erfolgt, wobei die Kontaktierung nur teilweise oder auf der gesamten für die Verbindung vorgesehenen Fläche erfolgt.

3. Verfahren zum Structural Health Monitoring mehrerer Klebefugen eines Schichtholzes nach Anspruch 2, **dadurch gekennzeichnet** als flächige leitfähige Elemente Kupferfolien oder Drahtgitter genutzt werden.

4. Verfahren zum Structural Health Monitoring mehrerer Klebefugen eines Schichtholzes nach einem der vorangehenden Ansprüche, **dadurch gekennzeichnet, dass** die Klebefugen derart kontaktiert werden, dass die elektrische Größe im Bereich der zu erwartenden maximalen Beanspruchung gemessen wird.

5. Anordnung zum Structural Health Monitoring nach Anspruch 1, umfassend Schichtholz mit mehreren Klebefugen zwischen jeweils zwei Fügepartnern (1), wobei im Klebstoff (2) der Klebefugen Carbon-Nano-Tubes enthalten sind, die in ihrer Lage bezogen auf die Fügepartner ausgerichtet sind, eine Messvorrichtung zur Bestimmung des elektrischen Widerstandes, und flächige leitfähige Elemente zur elektrischen Kontaktierung der Klebefugen mit der Messvorrichtung.

6. Anordnung nach Anspruch 5, **dadurch gekennzeichnet, dass** der Klebstoff 0,1 bis 10 M-% Carbon-Nano-Tubes enthält.

7. Anordnung nach einem der Ansprüche 5 oder 6, **dadurch gekennzeichnet, dass** die Carbon-Nano-Tubes folgende Durchmesser aufweisen: 10 bis 150 nm bei variabler Länge.

8. Anordnung nach einem der Ansprüche 5 bis 8, **dadurch gekennzeichnet, dass** der Klebstoff ausgewählt wird aus einer Gruppe die enthält: Melamin-Harnstoff-Formaldehyd-Klebstoff, Epoxidharz-, Ressorcin-, Isocyanat- Harnstoff-Melamin-Formaldehyd-, Harnstoff-Formaldehyd-, Kasein-, PvAC-, Ethylen-Vinyl-Acetat-, Polyolefinklebstoffe.

9. Anordnung nach einem der Ansprüche 5 bis 8, **dadurch gekennzeichnet, dass** die Klebeverbindung zwischen zwei Fügepartnern ausgeführt ist, als
-- vollflächige Klebeverbindung mit einem Klebstoff enthaltend Carbon-Nano-Tubes, oder
- teilflächige Klebeverbindung mit einem Klebstoff enthaltend Carbon-Nano-Tubes.

10. Anordnung nach einem der Ansprüche 5 bis 8, **dadurch gekennzeichnet, dass** die Klebeverbindung:
- mit mehreren Fügepartnern und einer vollflächig ausgeführten Klebefuge mit einem Klebstoff enthaltend Carbon-Nano-Tubes sowie nicht meßtechnisch wirksamen vollflächigen Klebefugen realisiert ist, oder
- mit mehreren Fügepartnern und einer Klebefuge, die zwei Teilflächen aus einem Klebstoff, enthaltend Carbon-Nano-Tubes, aufweist, sowie nicht meßtechnisch wirksamen vollflächigen Klebefugen realisiert ist, oder
- mit mehreren Fügepartnern und zwei teilflächig ausgeführten Klebefugen mit einem Klebstoff enthaltend Carbon-Nano-Tubes sowie nicht meßtechnisch wirksamen vollflächigen Klebefugen realisiert ist.

## Claims

1. A method for structural health monitoring of a plurality of adhesive joints of a laminated wood, wherein the adhesive (2) comprises carbon nanotubes in the adhesive joints between two join partners (1) each, with the following steps:
a) contacting the adhesive joints with a measuring device for measuring the electrical resistance of the adhesive joints,
b) measuring the electrical resistances of the adhesive joints,
c) comparing the measured electrical resistances of the adhesive joints to previously determined reference values,
d) determining the state of the adhesive joints to be monitored by means of the comparison from step c), **characterized in that**, prior to the setting or the curing, respectively, of the adhesive (2), the carbon nanotubes are aligned in their position with respect to the join partners (1) by means of a magnetic field, an electrical field or an electrical field with the presence of a magnetic field.

2. The method for structural health monitoring of a plurality of adhesive joints of a laminated wood according to claim 1, **characterized in that** in step a) the contacting of the adhesive joints takes place by means of flat, conductive elements, which are placed into the joint, wherein the contacting takes place only partially or on the entire surface, which is provided for the bond.

3. The method for structural health monitoring of a plurality of adhesive joints of a laminated wood according to claim 2, **characterized in that** copper foils or wire meshes are used as flat conductive elements.

4. The method for structural health monitoring of a plurality of adhesive joints of a laminated wood according to one of the preceding claims, **characterized in that** the adhesive joints are contacted in such a way that the electrical size is measured in the range of the expected maximum stress.

5. An arrangement for structural health monitoring according to claim 1 comprising laminated wood comprising a plurality of adhesive joints between two join partners (1) each, wherein carbon nanotubes, which are aligned in their position with respect to the join partners are comprised in the adhesive (2) of the adhesive joints, a measuring device for determining the electrical resistance, and flat conductive elements for electrically contacting the adhesive joints by means of the measuring device.

6. An arrangement according to claim 5, **characterized in that** the adhesive comprises between 0.1 and 10 % by mass of carbon nanotubes.

7. The arrangement according to one of claims 5 or 6, **characterized in that** the carbon nanotubes have the following diameters: 10 to 150 nm with variable length.

8. The arrangement according to one of claims 5 to 8, **characterized in that** the adhesive is chosen from a group, which comprises: melamine-urea-formaldehyde adhesive, epoxy resin-, resorcinol-, isocyanate- urea-melamine-formaldehyde, urea-formaldehyde, casein-, PvAC-, ethylene-vinyl-acetate-, polyolefin adhesives.

9. The arrangement according to one of claims 5 to 8, **characterized in that** the adhesive bond between two join partners is embodied as
- holohedral adhesive bond with an adhesive comprising carbon nanotubes, or
- partial adhesive bond with an adhesive comprising carbon nanotubes.

10. The arrangement according to one of claims 5 to 8, **characterized in that** the adhesive bond:
- is realized with a plurality of join partners and a holohedrally embodied adhesive joint with an adhesive comprising carbon nanotubes as well as holohedral adhesive joints, which are not effective by way of measurement techniques, or
- is realized with a plurality of join partners and an adhesive joint, which has two partial surfaces of an adhesive comprising carbon nanotubes, as well as holohedral adhesive joints, which are not effective by way of measurement techniques, or
- is realized with a plurality of join partners and two adhesive joints, which are embodied in a partial manner with an adhesive comprising carbon nanotubes as well as holohedral adhesive joints, which are not effective by way of measurement techniques.

## Revendications

1. Procédé pour le Structural Health Monitoring (surveillance de l'état des structures) de plusieurs joints adhésifs d'un bois stratifié, dans lequel l'adhésif (2) contient des nanotubes de carbone dans les joints adhésifs entre respectivement deux éléments de jointage associés (1), présentant les étapes suivantes :
a) mise en contact des joints adhésifs avec un dispositif de mesure pour mesurer la résistance électrique des joints adhésifs,
b) mesure des résistances électriques des joints adhésifs,
c) comparaison entre les résistances électriques mesurées des joints adhésifs et des grandeurs de référence préalablement déterminées,
d) détermination de l'état des joints adhésifs à surveiller à l'aide de la comparaison de l'étape c), **caractérisé en ce qu'**avant la prise ou le durcissement de l'adhésif (2), les nanotubes de carbone sont orientés concernant leur position par rapport aux éléments de jointage associés (1) au moyen d'un champ magnétique, d'un champ électrique ou d'un champ électrique en cas de présence d'un champ magnétique.

2. Procédé pour le Structural Health Monitoring de plusieurs joints adhésifs d'un bois stratifié selon la revendication 1, **caractérisé en ce qu'**à l'étape a), la mise en contact des joints adhésifs s'effectue au moyen d'éléments conducteurs en nappe insérés dans le joint, dans lequel la mise en contact s'effectue seulement partiellement ou sur l'ensemble de la surface prévue pour la liaison.

3. Procédé pour le Structural Health Monitoring de plusieurs joints adhésifs d'un bois stratifié selon la revendication 2, **caractérisé en ce que** l'on utilise des feuilles de cuivre ou du grillage en tant qu'éléments conducteurs en nappe.

4. Procédé pour le Structural Health Monitoring de plusieurs joints adhésifs d'un bois stratifié selon l'une des revendications précédentes, **caractérisé en ce que** les joints adhésifs sont mis en contact d'une façon telle, que la grandeur électrique est mesurée dans la zone attendue de sollicitation maximale.

5. Procédé pour le Structural Health Monitoring selon la revendication 1, comprenant du bois stratifié avec plusieurs joints adhésifs entre respectivement deux éléments de jointage associés (1), dans lequel des nanotubes de carbone sont contenus dans l'adhésif (2) des joints adhésifs, lesquels sont orientés concernant leur position par rapport aux éléments de jointage associés, un dispositif de mesure pour la détermination de la résistance électrique, et des éléments conducteurs en nappe pour la mise en contact électrique des joints adhésifs avec le dispositif de mesure.

6. Dispositif selon la revendication 5, **caractérisé en ce que** l'adhésif contient de 0,1 à 10% en masse de nanotubes de carbone.

7. Dispositif selon l'une des revendications 5 ou 6, **caractérisé en ce que** les nanotubes de carbone présentent les diamètres suivants : 10 à 150 mm avec une longueur variable.

8. Dispositif selon l'une des revendications 5 à 8, **caractérisé en ce que** l'adhésif est sélectionné dans un groupe comprenant : de l'adhésif de mélamine-urée-formaldéhyde, des adhésifs polyoléfines de résine époxy, résorcine, isocyanate, urée-mélamine-formaldéhyde, urée-formaldéhyde, caséine, PvAC, éthylène-vinyle-acétate.

9. Dispositif selon l'une des revendications 5 à 8, **caractérisé en ce que** la liaison adhésive entre deux éléments de jointage associés est réalisée en tant que :
- liaison adhésive sur toute la surface avec un adhésif contenant des nanotubes de carbone, ou
- liaison adhésive sur une surface partielle avec un adhésif contenant des nanotubes de carbone.

10. Dispositif selon l'une des revendications 5 à 8, **caractérisé en ce que** la liaison adhésive :
- est réalisée avec plusieurs éléments de jointage associés et un joint adhésif réalisé sur toute la surface avec un adhésif contenant des nanotubes de carbone ainsi que des joints adhésifs sur toute la surface non efficaces du point de vue de technique de mesure, ou
- avec plusieurs éléments de jointage associés et un joint adhésif qui présente deux surfaces partielles en un adhésif contenant des nanotubes de carbone, ainsi que des joints adhésifs sur toute la surface non efficaces du point de vue de technique de mesure, ou
- avec plusieurs éléments de jointage associés et deux joints adhésifs réalisés sur une surface partielle avec un adhésif contenant des nanotubes de carbone ainsi que des joints adhésifs sur toute la surface non efficaces du point de vue de technique de mesure.
